# EUROPÄISCHE PATENTSCHRIFT

(11) **EP 2 212 278 B1**
(45) Veröffentlichungstag und Bekanntmachung des Hinweises auf die Patenterteilung: **07.01.2015**
(21) Anmeldenummer: 08852108.3
(22) Anmeldetag: 14.11.2008
(51) Int. Cl.: C07C 231/02, C07C 233/47, A61K 8/44, A61Q 19/00

(54) **VERFAHREN ZUR HERSTELLUNG VON ACYLGLYCINATEN**
METHOD FOR PRODUCING ACYLGLYCINATES
PROCÉDÉ DE PRODUCTION D'ACYLGLYCINATES

(30) Priorität: 20.11.2007 DE 102007055265
(43) Veröffentlichungstag der Anmeldung: 04.08.2010
(60) Teilanmeldung: 10008258.5
(73) Patentinhaber: Clariant Finance (BVI) Limited, Road Town, Tortola (VG)
(72) Erfinder: KLUG, Peter, 63762 Grossostheim (DE); SCHERL, Franz-Xaver, 84508 Burgkirchen (DE)
(74) Vertreter: Paczkowski, Marcus
(86) Internationale Anmeldenummer: PCT/EP2008/009646
(87) Internationale Veröffentlichungsnummer: WO 2009/065530

(56) Entgegenhaltungen:
- EP-A- 1 672 055
- EP-A1- 1 314 717
- WO-A-96/39375
- WO-A-02/057217
- JP-A- H0 761 957
- US-A- 2 729 657

## Beschreibung

Die Erfindung betrifft ein Verfahren zur Herstellung von Acylglycinaten.

Acylglycinate der Formel (la) 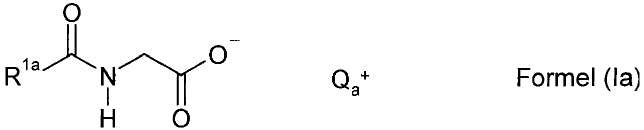 worin
R^{1a}-C(O) für eine lineare oder verzweigte, gesättigte Alkanoylgruppe mit 6 bis 30, vorzugsweise 8 bis 22, besonders bevorzugt 8 bis 18 Kohlenstoffatomen, oder für eine lineare oder verzweigte, ein- oder mehrfach ungesättigte Alkenoylgruppe mit 6 bis 30, vorzugsweise 8 bis 22 und besonders bevorzugt 12 bis 18 Kohlenstoffatomen steht, und
Qₐ⁺ für ein Kation steht, ausgewählt aus den Alkalimetallen Li⁺, Na⁺, K⁺, den Erdalkalimetallen Mg⁺⁺, Ca⁺⁺, aber auch für Al⁺⁺⁺ und/oder NH₄⁺, ein Monoalkylammonium-, Dialkylammonium-, Trialkylammonium- und/oder Tetraalkylammoniumion, wobei es sich bei den Alkylsubstituenten der Amine unabhängig voneinander um (C₁ - C₂₂)-Alkylreste oder (C₂ - C₁₀)-Hydroxyalkylreste handeln kann,
sind Tenside, die besonders in der Kosmetik für Gesichtsreinigungsrezepturen in Asien in Hautreinigungsprodukten geschätzt werden. Dies gilt insbesondere für Natrium- und Kaliumcocoylglycinat.

Die Tenside, insbesondere das Natrium- und das Kaliumcocoylglycinat, schäumen in leicht alkalischer Lösung exzellent und erzeugen ein angenehmes, nicht öliges Hautgefühl.

Im Gegensatz zu den entsprechenden N-Methylglycinderivaten, den sogenannten Sarkosinaten, weisen Glycinate bei der Herstellung jedoch problematische Eigenschaften auf. Wie in JP 8053693 beschrieben, lassen sich Acylglycinate in Wasser ohne zusätzliche Lösungsmittel nur in Reinheiten von knapp über 90 % erhalten und bilden bei der Schotten-Baumann-Reaktion sehr hochviskose Reaktionslösungen. Durch diese hohe Viskosität wird die Hydrolyse des zur Herstellung verwendeten Fettsäurechlorids begünstigt, die zu verminderter Reinheit des Acylglycinats führt. Die JP 8053693 schlägt daher bei der Herstellung von Acylglycinaten die Zugabe von Alkoholen wie Isopropanol, Isobutanol oder tert.-Butanol vor.

Diese Vorgehensweise ist jedoch wegen des Geruchs der Alkohole nicht vorteilhaft, weshalb die Alkohole aus der Reaktionsmischung auch nach Ansäuern und Phasentrennung wieder entfernt werden und das gewünschte Alkanoylglycinat nach Neutralisation meist als salzarme Qualität erhalten wird. Dieses Verfahren ist aufwendig und verursacht kochsalzhaltige Abwässer, die entsorgt werden müssen.

Es bestand daher die Aufgabe, ein Verfahren zur Herstellung von Acylglycinaten zur Verfügung zu stellen, das die oben genannten Nachteile nicht aufweist oder diese Nachteile zumindest abschwächt und insbesondere den Vorteil aufweist, dass es ohne Einsatz von organischen Lösemitteln erfolgen kann und Acylglycinate von hoher Reinheit liefert. Das Verfahren sollte auch die direkte Herstellung von Zusammensetzungen mit hohem Aktivgehalt an Acylglycinat und mit niedrigen Viskositäten ermöglichen.

Überraschenderweise wurde gefunden, dass diese Aufgabe gelöst wird, wenn die Acylglycinate durch Umsetzung von Glycin mit Fettsäurechlorid in Wasser und in Gegenwart einer basischen Alkaliverbindung, aber in Abwesenheit von organischen Lösungsmitteln, hergestellt werden, die Herstellung der Acylglycinate bei einer Temperatur von 30 - 35 °C durchgeführt wird, und der Anteil an Fettsäurechlorid enthaltend Acylgruppen mit 18 oder mehr Kohlenstoffatomen, bezogen auf die Gesamtmenge an eingesetztem Fettsäurechlorid, kleiner als 2,0 Gew.-% ist.

Gegenstand der Erfindung ist daher ein Verfahren zur Herstellung von Acylglycinaten der Formel (I) 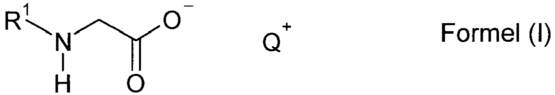 worin
R¹ die Bedeutung von R¹ aus dem eingesetzten C₈-₁₈-Cocoylchlorid R¹Cl besitzt, und
Q⁺ für ein Kation ausgewählt aus den Alkalimetallkationen Li⁺, Na⁺ und K⁺ steht,
dadurch gekennzeichnet, dass Glycin mit C₈₋₁₈-Cocoylchlorid R¹Cl, das einen Anteil an Fettsäurechlorid mit C₁₈-Acylgruppen, bezogen auf die Gesamtmenge an eingesetztem C₈₋₁₈-Cocoylchlorid, kleiner als 2,0 Gew.-% enthält, in Wasser und in Gegenwart einer basischen Alkaliverbindung, die Kationen Q⁺ ausgewählt aus Li⁺, Na⁺ und K⁺ liefert, aber in Abwesenheit von organischen Lösungsmitteln, bei 30 - 35 °C umgesetzt wird.

In WO 02/57217 A2 wird ein Verfahren zur Herstellung von Acylaminosäuren offenbart, bei dem man in einem Reaktor eine Mischung aus mindestens einer Aminosäure oder deren Salz und einer Alkaliquelle vorlegt und diese in einem Mischelement mit Fettsäurehalogeniden der Formel R¹COX, in der R¹ für einen Alkyl- oder Alkenylrest mit 6 bis 22 Kohlenstoffatomen und X für Chlor, Brom oder Jod steht, versetzt. Die in der WO 02/57217 A2 beschriebene Umsetzung mit dem Fettsäurehalogenid findet üblicherweise bei 20 - 25 °C statt. Die aus dem Verfahren resultierenden Produkte können in kosmetischen Produkten sowie in Wasch-, Spülund Reinigungsmitteln verwendet werden.

WO 96/39375 beschreibt ein Verfahren zur Herstellung von Alkalimetall N-Acylaminosäuren, wobei Fettsäure mit Aminosäure oder Alkalimetall-Aminosäure umgesetzt wird.

EP 1672055 A1 offenbart ein Verfahren zur Herstellung von Salzen neutraler Aminosäuren, die am Stickstoff durch langkettige Acylgruppen substituiert sind. Im Verfahren werden die 3 Komponenten (a) langkettige Fettsäure, (b) neutrale Aminosäure und (c) mindestens eine Substanz ausgewählt aus Natriumhydroxid und Kaliumhydroxid gemischt und die Mischung auf eine Temperatur von 150 - 190 °C erhitzt, wobei das in der Kondensationsreaktion entstehende Wasser kontinuierlich entfernt wird.

In EP 1314717 A1 wird ein Verfahren zur Herstellung von neutralen Aminosäuren, die am Stickstoff durch eine langkettige Acylgruppe substituiert sind, beschrieben. Im Verfahren wird eine neutrale Aminosäure und ein gesättigtes oder ungesättigtes Fettsäurechlorid mit 8 bis 22 Kohlenstoffatomen umgesetzt, wobei die Reaktion in einer Mischung aus Wasser und einem oder mehreren hydrophilen organischen Lösungsmitteln ausgewählt aus der Gruppe bestehend aus Aceton, Acetonitril, einem sekundären Alkohol mit 3 oder 4 Kohlenstoffatomen und einem tertiären Alkohol mit 4 Kohlenstoffatomen in Gegenwart einer Base durchgeführt wird.

Im Rahmen der vorliegenden Erfindung werden die Gruppen R¹ aus Formel (I) auch als "Acylgruppen" bezeichnet.

Die im erfindungsgemäßen Verfahren verwendeten Fettsäurechloride mit niedrigem Anteil an langkettigen Acylgruppen können nach dem Fachmann bekannten Methoden erhalten werden, z. B. durch Destillation aus gewöhnlichen Fettsäurechloriden.

Als basische Alkaliverbindungen werden vorzugsweise Carbonate oder Hydroxide, vorzugsweise Hydroxide, der Alkalimetallkationen Li⁺, Na⁺ oder K⁺ oder Mischungen davon eingesetzt. Besonders bevorzugt sind NaOH und/oder KOH, insbesondere bevorzugt ist NaOH.

Das erfindungsgemäße Verfahren wird vorzugsweise bei einem pH-Wert von 9 bis 13, besonders bevorzugt 12 bis 13, durchgeführt.

Weiterhin bevorzugt wird das erfindungsgemäße Verfahren derart durchgeführt, dass Glycin und Fettsäurechlorid in equimolaren Mengen eingesetzt werden. Besonders bevorzugt wird das Fettsäurechlorid bezogen auf Glycin in leichtem Unterschuss eingesetzt. Das molare Verhältnis von Glycin zu Fettsäurechlorid R¹Cl ist insbesondere bevorzugt von 1,1 : 1,0 bis 1,0 : 1,0 und außerordentlich bevorzugt von 1,05 : 1,0 bis 1,0 : 1,0.

In dem erfindungsgemäßen Verfahren werden C₈₋₁₈-Cocoylchloride, die einen Anteil an Fettsäurechlorid mit C₁₈-Acylgruppen kleiner 2,0 Gew.-%, bezogen auf die Gesamtmenge an eingesetztem Fettsäurechlorid, enthalten, in Gegenwart einer basischen Alkaliverbindung, die Kationen Q⁺ ausgewählt aus Li⁺, Na⁺ und K⁺, vorzugsweise ausgewählt aus Na⁺ und K⁺ und besonders bevorzugt Na⁺, liefert, umgesetzt. Bei den basischen Alkaliverbindungen handelt es sich vorzugsweise um Carbonate oder Hydroxide, vorzugsweise um Hydroxide, der Alkalimetallkationen Li⁺, Na⁺ oder K⁺ oder Mischungen davon, besonders bevorzugt um NaOH oder KOH und insbesondere bevorzugt um NaOH.

In einer besonders bevorzugten Ausführungsform des erfindungsgemäßen Verfahrens werden Cocoylchloride mit C₈₋₁₈-Acylgruppen, und wobei der Anteil an Fettsäurechloriden mit C₁₈-Acylgruppen, bezogen auf die Gesamtmenge an eingesetztem Cocoylchlorid, kleiner als 2,0 und bevorzugt kleiner als 1,0 Gew.-% ist, in Gegenwart einer basischen Alkaliverbindung, die Na⁺-Kationen liefert, umgesetzt. Bei den basischen Alkaliverbindungen handelt es sich vorzugsweise um Na₂CO₃ oder NaOH und besonders bevorzugt um NaOH.

In einer insbesondere bevorzugten Ausführungsform des erfindungsgemäßen Verfahrens werden Cocoylchloride mit C₈₋₁₈-Acylgruppen, und wobei der Anteil an Fettsäurechloriden mit C₈- und C₁₀-Acylgruppen zusammen größer als 5,0 Gew.-%, bevorzugt von 10,0 bis 14,0 Gew.-%, der Anteil an Fettsäurechloriden mit C₁₂-Acylgruppen von 50,0 bis 72,0 Gew.-%, und der Anteil an Fettsäurechloriden mit C₁₈-Acylgruppen kleiner als 2,0 und bevorzugt kleiner als 1,0 Gew.-%, jeweils bezogen auf die Gesamtmenge an eingesetztem Cocoylchlorid, ist, in Gegenwart einer basischen Alkaliverbindung, die Na⁺-Kationen liefert, umgesetzt. Bei den basischen Alkaliverbindungen handelt es sich vorzugsweise um Na₂CO₃ oder NaOH und besonders bevorzugt um NaOH.

Das erfindungsgemäße Verfahren wird vorzugsweise derart ausgeführt, dass Glycin in Wasser in Gegenwart der basischen Alkaliverbindung wie beispielsweise NaOH vorgelegt wird und das Fettsäurechlorid bei 30 bis 35 °C zugegeben wird. Die Zugabe des Fettsäurechlorids erfolgt dabei vorzugsweise langsam unter Rühren.

Durch das erfindungsgemäße Verfahren können höher konzentrierte, salzhaltige Glycinatlösungen mit niedrigem Gehalt an Nebenprodukten (wie beispielsweise

Fettsäuresalz) hergestellt werden, die niedrigviskos und dementsprechend einfach zu handhaben sind und darüber hinaus kosteneffektiv sind, da kein Separationsschritt durchgeführt werden muss. Außerdem wird dabei kein organisches Reaktionslösungsmittel benötigt, das wieder abgetrennt und gegebenenfalls entsorgt werden muss.

Durch das erfindungsgemäße Verfahren können Zusammensetzungen hergestellt werden enthaltend
a) Acylglycinate der Formel (I) 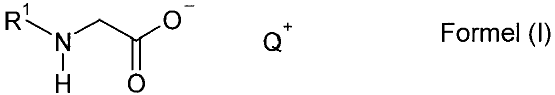 worin
   R¹ die Bedeutung von R¹ aus dem im erfindungsgemäßen Verfahren eingesetzten C₈₋₁₈-Cocoylchlorid R¹Cl besitzt und
   Q⁺ für ein Kation ausgewählt aus den Alkalimetallkationen Li⁺, Na⁺ und K⁺, vorzugsweise ausgewählt aus Na⁺ und K⁺ und besonders bevorzugt für Na⁺, steht,
   in Mengen von 21,0 - 28,0 Gew.-%, bezogen auf die gesamte Zusammensetzung,
   und wobei der Anteil an Acylglycinaten der Formel (I) enthaltend Acylgruppen R¹ mit 18 Kohlenstoffatomen, bezogen auf die Gesamtmenge an Acylglycinaten der Formel (I), kleiner als 2,0, bevorzugt kleiner als 1,8, Gew.-% ist,
b) ein oder mehrere Substanzen Q⁺Cl⁻, worin Q⁺ die Bedeutung von Q⁺ aus Formel (I) hat, in Mengen größer oder gleich 1,0 Gew.-%, bezogen auf die gesamte Zusammensetzung,
c) ein oder mehrere Fettsäuresalze der Formel (II) 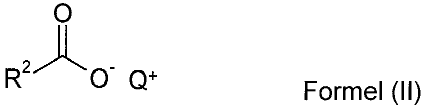 worin
   R²CO die Bedeutung von R¹ aus Formel (I) und
   Q⁺ die Bedeutung von Q⁺ aus Formel (I) hat,
   in Mengen kleiner als 2,0 Gew.-%, vorzugsweise in Mengen größer als 0,01 Gew.-% und kleiner als 2,0 Gew.-% und besonders bevorzugt in Mengen größer als 0,1 Gew.-% und kleiner als 2,0 Gew.-%, bezogen auf die gesamte Zusammensetzung, und
d) Wasser,
e) aber keine organischen Lösungsmittel.
   Q⁺Cl⁻ wird auch als QCl bezeichnet, z.B. Na⁺Cl⁻ als NaCl.

In den Verbindungen der Formeln (I) und (II) sowie in Q⁺Cl⁻ ist Q⁺ ausgewählt aus Li⁺, Na⁺ und K⁺, vorzugsweise ausgewählt aus Na⁺ und K⁺ und besonders bevorzugt steht Q⁺ für Na⁺.

Besonders bevorzugt sind Zusammensetzungen enthaltend Natriumcocoylglycinat, wobei der Cocoylschnitt nur geringe Mengen an C₁₈-Acylgruppen aufweist.

Besonders bevorzugt enthalten die Zusammensetzungen als Komponente a) Natriumcocoylglycinat mit C₈₋₁₈-Acylgruppen in Mengen von 21,0 - 28,0 Gew.-% und vorzugsweise in Mengen von 23,0 - 27,0 Gew.-%, bezogen auf die gesamte Zusammensetzung, und wobei der Anteil an Acylglycinaten der Formel (I) mit C₁₈-Acylgruppen kleiner als 2,0, bevorzugt kleiner als 1,8, und besonders bevorzugt kleiner als 1,0 Gew.-%, bezogen auf die Gesamtmenge an Natriumcocoylglycinat, ist. In dieser Ausführungsform ist Q⁺ in Q⁺Cl⁻ und in dem Fettsäuresalz der Formel (II) Na⁺.

Insbesondere bevorzugt enthalten die Zusammensetzungen als Komponente a) Natriumcocoylglycinat mit C₈₋₁₈-Acylgruppen in Mengen von 21,0 - 28,0 Gew.-% und vorzugsweise in Mengen von 23,0 - 27,0 Gew.-%, bezogen auf die gesamte Zusammensetzung, und wobei der Anteil an Acylglycinaten der Formel (I) mit C₈- und C₁₀-Acylgruppen zusammen größer als 5,0 Gew.-%, bevorzugt von 10,0 bis 14,0 Gew.-%, der Anteil an Acylglycinaten der Formel (I) mit C₁₂-Acylgruppen von 50,0 bis 72,0 Gew.-%, und der Anteil an Acylglycinaten der Formel (I) mit C₁₈-Acylgruppen kleiner als 2,0, bevorzugt kleiner als 1,8, und besonders bevorzugt kleiner als 1,0 Gew.-%, jeweils bezogen auf die Gesamtmenge an Natriumcocoylglycinat, ist. In dieser Ausführungsform ist Q⁺ in Q⁺Cl⁻ und in dem Fettsäuresalz der Formel (II) Na⁺.

Ausserordentlich bevorzugt ist die Reinheit der in den Zusammensetzungen enthaltenen Natriumcocoylglycinate 97 % oder größer. Diese Reinheit ist auf die Summe an Fettsäuresalz der Formel (II) und Acylglycinat der Formel (I) bezogen. Sie berechnet sich nach der Formel "Reinheit der Natriumcocoylglycinate = [Menge an Natriumcocoylglycinat : (Menge an Natriumcocoylglycinat + Menge an Fettsäuresalz)]".

Die Zusammensetzungen können beispielsweise vorteilhaft für kosmetische Anwendungen verwendet werden.

Für die kosmetischen Anwendungen sehr vorteilhaft ist, dass die Zusammensetzungen Q⁺Cl⁻ in Mengen von 1,0 bis 8,0 Gew.-% enthalten, so dass eine gesonderte Zugabe von Q⁺Cl⁻ als Viskositätsregulierer in den Endformulierungen entfallen kann.

Bevorzugt enthalten die Zusammensetzungen daher 1,0 bis 8,0, bevorzugt 2,0 bis 7,0 und besonders bevorzugt 4,0 bis 6,0 Gew.-% Q⁺Cl⁻, bezogen auf die gesamte Zusammensetzung.

Ein weiterer Vorteil der Zusammensetzungen ist, dass diese nur geringe Mengen an Fettsäuresalz enthalten.

Weiter bevorzugt enthalten die Zusammensetzungen daher weniger als 1,8, bevorzugt weniger als 1,5 und besonders bevorzugt weniger als 1,0 Gew.-% an Fettsäuresalz der Formel (II), bezogen auf die gesamte Zusammensetzung. Dabei ist die untere Grenze vorzugsweise jeweils größer 0,01 Gew.-% und besonders bevorzugt jeweils größer 0,1 Gew.-%, bezogen auf die gesamte Zusammensetzung.

Die Zusammensetzungen sind flüssig.

Sehr vorteilhaft ist auch die relativ geringe Viskosität und damit eine einfache Handhabung der Zusammensetzungen.

Weiter bevorzugt weisen die Zusammensetzungen Viskositäten bei 35 °C von kleiner als 5.000 mPa·s, bevorzugt von 400 bis 2.000 mPa·s und besonders bevorzugt von 500 bis 1.000 mPa·s auf.

Bei dem für die Bestimmung der Viskosität verwendeten Gerät handelt es sich um ein Rotameter der Firma Thermo Haake (Viskotester 550). Als Spindel wurde MV-DIN (45,3 Umdrehungen / Minute) verwendet.

Die Zusammensetzungen enthalten keine organischen Lösungsmittel, wie beispielsweise niedere Alkohole, Diole oder andere Lösungsmittel.

Besonders bevorzugt bestehen die Zusammensetzungen aus den Komponenten a), b), c) und d).

Weiter besonders bevorzugt bestehen die Zusammensetzungen aus den Komponenten a), b), c), d) und H₂NCH₂COO⁻ Q⁺, wobei Q⁺ für ein Kation ausgewählt aus Li⁺, Na⁺ und K⁺, vorzugsweise ausgewählt aus Na⁺ und K⁺ und besonders bevorzugt für Na⁺, steht. In diesen Zusammensetzung ist die Verbindung H₂NCH₂COO⁻ Q⁺, bezogen auf die gesamte Zusammensetzung, vorzugsweise von 0,01 bis 1,0 und besonders bevorzugt von 0,05 bis 0,5 Gew.-%, enthalten.

Die Zusammensetzungen sind in vorteilhafter Weise zur Herstellung kosmetischer Zubereitungen geeignet.

Besonders vorteilhaft dabei ist, dass die Zusammensetzungen wie aus dem erfindungsgemäßen Verfahren erhalten, d. h. ohne weitere Aufarbeitung oder Aufreinigung, verwendet werden können.

Die Zusammensetzungen sind zudem in vorteilhafter Weise als Tenside in kosmetischen Zubereitungen geeignet.

Die Zusammensetzungen können auch hierbei direkt wie aus dem erfindungsgemäßen Verfahren erhalten verwendet werden.

Die nachfolgenden Beispiele und Anwendungen sollen die Erfindung näher erläutern.

Bei allen Prozentangaben handelt es sich um Gewichtsprozent (Gew.-%).

### Cocoylchlorid (A): Cocosschnitt mit reduziertem Anteil an C₁₆ und C₁₈ Spezifikation des verwendeten Cocoylchlorids:

| | |
|---|---|
| C₈/C₁₀: | 10,0-14,0% |
| C₁₂: | 60,0-62,0% |
| C₁₄: | 19,0-24,0% |
| C₁₆: | 3,0-10,0% |
| C₁₈: | < 2,0 % |

### Beispiel 1

37,8 g (0,504 mol) Glycin wird in 276 g VE-Wasser (vollentsalztes Wasser) unter Rühren gelöst und der pH-Wert (telquel) mit Natronlauge (33 %) auf 12-13 eingestellt. Anschließend wird unter Rühren auf 30 - 35 °C aufgeheizt und 106,4 g (0,478 mol) Cocoylchlorid (A) innerhalb 6 Stunden bei 30 - 35 °C unter Kühlung des Reaktionsgemisches zudosiert. Der pH-Wert wird durch gleichzeitige Dosierung von Natronlauge (33 %) bei 12-13 gehalten. Gegen Ende der Dosierung des Cocoylchlorids lässt man den pH-Wert auf 9,5 - 10,5 abfallen. Zur Vervollständigung der Reaktion wird noch 2 Stunden bei pH 9,5 - 10,5 gerührt.

Das erhaltene Produkt hat folgende Eigenschaften: flüssig, opal, Trockenrückstand (1 h, 140 °C): 31,0 %, Glycinsalz (HPLC): 0,6 %, Fettsäuresalz (HPLC): 0,6 %, Viskosität (35 °C): 756 mPa·s, NaCl (Titration): 5,3 %, Aktivgehalt: 24,5 %

Die Berechnung der Gewichtsmenge an Acylglycinat in den Zusammensetzungen erfolgt durch die Formel "Gewichtsmenge an Acylglycinat = Trockenrückstand - Fettsäuresalz - Glycinsalz - Q⁺Cl⁻". Der Wert wird im Rahmen der vorliegenden Erfindung als "Aktivgehalt" bezeichnet.

### Vergleichsbeispiel 2 : höhere Reaktionstemperatur

37,8 g (0,504 mol) Glycin wird in 276 g VE-Wasser unter Rühren gelöst und der pH-Wert (telquel) mit Natronlauge (33 %) auf 12-13 eingestellt. Anschließend wird unter Rühren auf 45 - 50 °C aufgeheizt und 106,4 g (0,478 mol) Cocoylchlorid (A) innerhalb 6 Stunden bei 45 - 50 °C zudosiert. Der pH-Wert wird durch gleichzeitige Dosierung von Natronlauge (33 %) bei 12-13 gehalten. Nach ca. 1/6 der Dosierung des Cocoylchlorids wird der Ansatz gestoppt, da bereits die berechnete Menge an Natronlauge (33 %) des gesamten Ansatzes verbraucht ist.

Der Ansatz ist zweiphasig (Fettsäuresalz). Die erhöhte Temperatur führt vorwiegend zur Hydrolyse des Cocoylchlorids.

### Vergleichsbeispiel 3 : niedrigere Reaktionstemperatur

37,8 g (0,504 mol) Glycin wird in 276 g VE-Wasser unter Rühren gelöst und der pH-Wert (telquel) mit Natronlauge (33 %) auf 12-13 eingestellt. Anschließend wird unter Rühren auf 25 - 30 °C aufgeheizt und 106,4 g (0,478 mol) Cocoylchlorid (A) innerhalb 6 Stunden bei 25 - 30 °C unter Kühlung des Reaktionsgemisches zudosiert. Der pH-Wert wird durch gleichzeitige Dosierung von Natronlauge (33 %) bei 12-13 gehalten. Gegen Ende der Dosierung des Cocoylchlorids lässt man den pH-Wert auf 9,5 - 10,5 abfallen. Zur Vervollständigung der Reaktion wird noch 2 Stunden bei pH 9,5 -10,5 gerührt.

Das erhaltene Produkt hat folgende Eigenschaften: flüssig, trüb, Trockenrückstand (1h, 140 °C): 30,9 %, Glycinsalz (HPLC): 1,3 %, Fettsäuresalz (HPLC): 2,4 %, Viskosität: nicht bestimmt, NaCl (Titration): 5,3%, Aktivgehalt: 21,9 %

### Beispiel 4 : höherer Glycinüberschuß

37,8 g (0,504 mol) Glycin wird in 250 g VE-Wasser unter Rühren gelöst und der pH-Wert (telquel) mit Natronlauge (33 %) auf 12-13 eingestellt. Anschließend wird unter Rühren auf 30 - 35 °C aufgeheizt und 95,2 g (0,428 mol) Cocoylchlorid (A) innerhalb 6 Stunden bei 30 - 35 °C unter Kühlung des Reaktionsgemisches zudosiert. Der pH-Wert wird durch gleichzeitige Dosierung von Natronlauge (33 %) bei 12-13 gehalten. Gegen Ende der Dosierung des Cocoylchlorids lässt man den pH-Wert auf 9,5 - 10,5 abfallen. Zur Vervollständigung der Reaktion wird noch 2 Stunden bei pH 9,5 - 10,5 gerührt.

Das erhaltene Produkt hat folgende Eigenschaften: flüssig, opal, Trockenrückstand (1h, 140 °C): 31,3 %, Glycinsalz (HPLC): 1,3%, Fettsäuresalz (HPLC): 0,2 %, Viskosität (35 °C): 980 mPa·s, NaCl (Titration): 5,1 %, Aktivgehalt: 24,7 %

### Beispiel 5: höhere Konzentration

37,8 g (0,504 mol) Glycin wird in 246 g VE-Wasser unter Rühren gelöst und der pH-Wert (telquel) mit Natronlauge (33 %) auf 12-13 eingestellt. Anschließend wird unter Rühren auf 30 - 35 °C aufgeheizt und 106,4 g (0,478 mol) Cocoylchlorid (A) innerhalb 6 Stunden bei 30 - 35 °C unter Kühlung des Reaktionsgemisches zudosiert. Der pH-Wert wird durch gleichzeitige Dosierung von Natronlauge (33 %) bei 12-13 gehalten. Gegen Ende der Dosierung des Cocoylchlorids lässt man den pH-Wert auf 9,5 - 10,5 abfallen. Zur Vervollständigung der Reaktion wird noch 2 Stunden bei pH 9,5 - 10,5 gerührt.

Das erhaltene Produkt hat folgende Eigenschaften: flüssig, opal, Trockenrückstand (1 h, 140 °C): 32,7 %, Glycinsalz (HPLC): 0,8 %, Fettsäuresalz (HPLC): 1,3 %, Viskosität (35 °C): 4.500 mPa·s, NaCl (Titration): 5,6 %, Aktivgehalt: 25,0 %

### Vergleichsbeispiel 6: salzfreie Herstellung in Propanol-2/Wasser

94,5 g (1,26 mol) Glycin wird in 400 g VE-Wasser und 276 g Propanol-2 unter Rühren gelöst und der pH-Wert (telquel) mit Natronlauge (33 %) auf 12-13 eingestellt. Anschließend werden unter Rühren bei 25 - 27 °C 283,9 g (1,24 mol) Cocoylchlorid (A) innerhalb 6 Stunden unter Kühlung des Reaktionsgemisches zudosiert. Der pH-Wert wird durch gleichzeitige Dosierung von Natronlauge (33 %) bei 12-13 gehalten. Zur Vervollständigung der Reaktion wird noch 2 Stunden bei pH 12-13 gerührt. Das erhaltene Produkt wird mit konz. Salzsäure auf pH 1 gestellt. Nach Abschalten des Rührers beginnt sofort die Phasentrennung. Die wässrige Phase wird abgetrennt und die organische Phase mit Natronlauge (33 %) auf pH 4,6 eingestellt. Nach Zugabe von 740 g Wasser wird mit Natronlauge (33 %) auf pH 7,0 gestellt. Anschließend wird bei vermindertem Druck (ca. 140-160 mbar) und 55 °C Propanol-2/Wasser abdestilliert bis der Propanol-2-Gehalt im Produkt unter 1 % gefallen ist. Mit Hilfe von Wasser und Natronlauge wird ein Feststoffgehalt von 30 % und ein pH-Wert von 10,0 eingestellt.

Das Produkt hat folgende Eigenschaften: flüssig, klar, Trockenrückstand (1 h, 140 °C): 30,3 %, Glycinsalz (HPLC): <0,1 %, Fettsäuresalz (HPLC): 1,0 %, Propanol-2 (GC): <0,1 %, Chlorid (Titration): 0,35 %, Viskosität: nicht bestimmt, NaCl (Titration): 0,6 %, Aktivgehalt: 28,7 %

### Vergleichsbeispiel 7: Cocosschnitt mit erhöhtem Anteil an C_{16/18}, hydriert

Verteilung des verwendeten Cocoylchlorids (B) :

| | |
|---|---|
| C₁₂: | 55,6% |
| C₁₄: | 23,0% |
| C₁₆: | 11,1% |
| C₁₈ gesättigt: | 10,3% |

36,0 g (0,480 mol) Glycin wird in 280 g VE-Wasser unter Rühren gelöst und der pH-Wert (telquel) mit Natronlauge (33 %) auf 12-13 eingestellt. Anschließend wird unter Rühren auf 30 - 35 °C aufgeheizt und 109,6 g (0,456 mol) Cocoylchlorid (B) innerhalb 6 Stunden bei 30 - 35 °C unter Kühlung des Reaktionsgemisches zudosiert. Der pH-Wert wird durch gleichzeitige Dosierung von Natronlauge (33 %) bei 12-13 gehalten. Im Laufe der Dosierung des Cocoylchlorids wird der Ansatz immer zähflüssiger bis er nicht mehr rührbar ist. Durch Zugabe von 110 g Wasser und anschließende Erhöhung der Reaktionstemperatur auf 40 °C bleibt der Ansatz einigermaßen rührbar. Gegen Ende der Dosierung des Cocoylchlorids lässt man den den pH-Wert auf 9,5 - 10,5 abfallen. Zur Vervollständigung der Reaktion wird noch 2 Stunden bei pH 9,5 - 10,5 gerührt.

Das erhaltene Produkt hat folgende Eigenschaften: flüssig, trüb, Trockenrückstand (1 h, 140 °C): 25,8 %, Glycinsalz (HPLC): 0,8 %, Fettsäuresalz (HPLC): 1,2 %, Viskosität: nicht bestimmt, NaCl (Titration): 4,1 %, Aktivgehalt: 19,7 %

Die beschriebenen Beispiele belegen, dass hochkonzentrierte Alkalisalzglycinatlösungen mit > 22,0 %, vorzugsweise > 23,0 % und besonders bevorzugt > 24,0 % Aktivgehalt durch Verwendung eines C₁₈-armen Cocosfettsäureschnitts und Reaktion in Wasser ohne zusätzliche Lösungsmittel bei 30 - 35 °C erhalten werden können (Beispiel 1, Beispiel 4, Beispiel 5). In Beispiel 1 konnte eine Reinheit der Glycinatlösung von 97,6 % Acylglycinat bezogen auf die Summe aus Fettsäuresalz und Acylglycinat erreicht werden. Dagegen führen höhere Reaktionstemperaturen (Vergleichsbeispiel 2) zu stark erhöhten Fettsäuresalzgehalten. Niedrigere Reaktionstemperaturen (Vergleichsbeispiel 3) führen ebenfalls zu unerwünscht hohen Anteilen an Fettsäuresalzen. Bei Verwendung C₁₆/C₁₈-reicher Fettsäurechloride (Vergleichsbeispiel 7) werden unerwünscht niedrige Aktivgehalte erhalten.

## Patentansprüche

1. Verfahren zur Herstellung von Acylglycinaten der Formel (I) 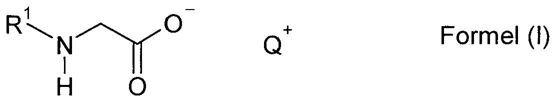 worin
R¹ die Bedeutung von R¹ aus dem eingesetzten C₈₋₁₈-Cocoylchlorid R¹Cl besitzt, und
Q⁺ für ein Kation ausgewählt aus den Alkalimetallkationen Li⁺, Na⁺ und K⁺ steht,
**dadurch gekennzeichnet, dass** Glycin mit C₈₋₁₈-Cocoylchlorid R¹Cl, das einen Anteil an Fettsäurechlorid mit C₁₈-Acylgruppen, bezogen auf die Gesamtmenge an eingesetztem C₈₋₁₈-Cocoylchlorid, kleiner als 2,0 Gew.-% enthält, in Wasser und in Gegenwart einer basischen Alkaliverbindung, die Kationen Q⁺ ausgewählt aus Li⁺, Na⁺ und K⁺ liefert, aber in Abwesenheit von organischen Lösungsmitteln, bei 30 - 35 °C umgesetzt wird.

2. Verfahren nach Anspruch 1, **dadurch gekennzeichnet, dass** als basische Alkaliverbindungen Carbonate oder Hydroxide der Alkalimetallkationen Li⁺, Na⁺ oder K⁺ oder Mischungen davon eingesetzt werden.

3. Verfahren nach Anspruch 1 oder 2, **dadurch gekennzeichnet, dass** es bei einem pH-Wert von 9 bis 13 durchgeführt wird.

4. Verfahren nach einem oder mehreren der Ansprüche 1 bis 3, **dadurch gekennzeichnet, dass** das molare Verhältnis von Glycin zu Fettsäurechlorid R¹Cl von 1,1 : 1,0 bis 1,0 : 1,0 ist.

## Claims

1. A method for producing acylglycinates of the formula (I) 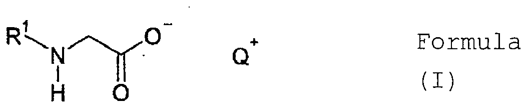 in which
R¹ has the meaning of R¹ from the C₈₋₁₈ cocoyl chloride R¹Cl used, and
Q⁺ is a cation selected from the alkali metal cations Li⁺, Na⁺ and K⁺, z
wherein glycine is reacted with C₈₋₁₈ cocoyl chloride R¹Cl containing a proportion of fatty acid chloride having C₁₈ acyl groups, based on the total amount of C₈₋₁₈ cocoyl chloride used, of less than 2.0% by weight, in water and in the presence of a basic alkali metal compound which provides cations Q⁺ selected from Li⁺, Na⁺ and K⁺, but in the absence of organic solvents, at 30 - 35°C.

2. A method as claimed in claim 1, wherein carbonates or hydroxides of the alkali metal cations Li⁺, Na⁺ or K⁺ or mixtures thereof are used as basic alkali metal compounds.

3. A method as claimed in claim 1 or 2, wherein it is carried out at a pH of from 9 to 13.

4. A method as claimed in one or more of claims 1 to 3, wherein the molar ratio of glycine to fatty acid chloride R¹Cl is from 1.1:1.0 to 1.0:1.0.

## Revendications

1. Procédé de fabrication de glycinates d'acyle de formule (I) 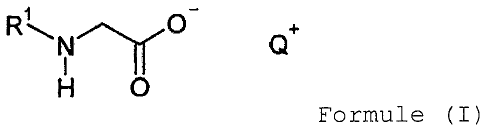 dans laquelle
R¹ a la signification de R¹ dans le chlorure de cocoyle en C₈-C₁₈ R¹Cl utilisé et
Q⁺ représente un cation choisi parmi les cations de métaux alcalins Li⁺, Na⁺ et K⁺,
**caractérisé en ce que** de la glycine est mise en réaction avec un chlorure de cocoyle en C₈-C₁₈ R¹Cl, qui contient une proportion de chlorure d'acide gras contenant des groupes acyle en C₁₈, par rapport à la quantité totale de chlorure de cocoyle en C₈-C₁₈ utilisé, inférieure à 2,0 % en poids, dans de l'eau et en présence d'un composé alcalin basique qui fournit des cations Q⁺ choisis parmi Li⁺, Na⁺ et K⁺, mais en l'absence de solvants organiques, à une température de 30 à 35 °C.

2. Procédé selon la revendication 1, **caractérisé en ce que** des carbonates ou des hydroxydes des cations de métaux alcalins Li⁺, Na⁺ ou K⁺ ou leurs mélanges sont utilisés en tant que composés alcalins basiques.

3. Procédé selon la revendication 1 ou 2, **caractérisé en ce qu'**il est réalisé à un pH de 9 à 13.

4. Procédé selon une ou plusieurs des revendications 1 à 3, **caractérisé en ce que** le rapport molaire entre la glycine et le chlorure d'acide gras R¹Cl est de 1,1:1,0 à 1,0:1,0.
